# EUROPÄISCHE PATENTSCHRIFT

(11) **EP 2 780 063 B1**
(45) Veröffentlichungstag und Bekanntmachung des Hinweises auf die Patenterteilung: **21.08.2019**
(21) Anmeldenummer: 12786875.0
(22) Anmeldetag: 30.10.2012
(51) Int. Cl.: A61M 5/32, A61M 5/31, A61M 5/24, A61M 5/46, A61M 5/34, A61M 5/315

(54) **GLASSPRITZENSEITIGES MONTAGEHILFSELEMENT, VERFAHREN ZUM BEFESTIGEN EINES GLASSPRITZENSEITIGEN MONTAGEHILFSELEMENTS UND VERFAHREN ZUM HERSTELLEN EINES GLASSPRITZENSEITIGEN MONTAGEHILFSELEMENTS SOWIE ANORDNUNG AUS EINER GLASSPRITZE UND AUS EINEM MONTAGEHILFSELEMENT**
GLASS SYRINGE-SIDE ASSEMBLY AID ELEMENT, METHOD FOR FASTENING A GLASS SYRINGE-SIDE ASSEMBLY AID ELEMENT AND METHOD FOR PRODUCING A GLASS SYRINGE-SIDE ASSEMBLY AID ELEMENT, AS WELL AS AN ARRANGEMENT CONSISTING OF A GLASS SYRINGE AND AN ASSEMBLY AID ELEMENT
AUXILIAIRE DE MONTAGE CÔTÉ SERINGUE EN VERRE, PROCÉDÉ DE FIXATION D'UN AUXILIAIRE DE MONTAGE CÔTÉ SERINGUE EN VERRE ET PROCÉDÉ DE FABRICATION D'UN AUXILIAIRE DE MONTAGE CÔTÉ SERINGUE EN VERRE ET ENSEMBLE FORMÉ PAR UNE SERINGUE EN VERRE ET UN AUXILIAIRE DE MONTAGE

(30) Priorität: 15.11.2011 DE 102011055389
(43) Veröffentlichungstag der Anmeldung: 24.09.2014
(73) Patentinhaber: Gerresheimer Regensburg GmbH, 93047 Regensburg (DE)
(72) Erfinder: PFRANG, Jürgen, 93183 Kallmünz (DE)
(74) Vertreter: Hannke, Christian
(86) Internationale Anmeldenummer: PCT/EP2012/071459
(87) Internationale Veröffentlichungsnummer: WO 2013/072182

(56) Entgegenhaltungen:
- EP-A1- 1 462 134
- WO-A1-01/07105
- WO-A1-93/10838
- WO-A1-2004/108194
- US-A- 3 605 743
- US-A- 4 518 387

## Beschreibung

Die Erfindung betrifft eine Anordnung aus einer Glasspritze und aus einem Montagehilfselement, gemäß dem Oberbegriff des Anspruchs 1.

Ferner betrifft die Erfindung ein Verfahren zum Befestigen des glasspritzenseitigen Montagehilfselements der Anordnung nach einem der Ansprüche 1 bis 4 an einer Glasspritze nach einem der Ansprüche 1 bis 4, gemäß dem Oberbegriff des Anspruchs 5.

Außerdem betrifft die Erfindung ein Verfahren zum Herstellen des glasspritzenseitigen Montagehilfselements der Anordnung nach einem der Ansprüche 1 bis 4, gemäß dem Oberbegriff des Anspruchs 6.Gattungsgemäße Glasspritzen sind insbesondere als Ganzglasspritzen weit verbreitet. Derartige Glasspritzen sind historisch bedingt, aber auch herstellungsbedingt als Stand-Alone-Device konzipiert. Ein Einsatz im Zusammenhang mit anderen medizinischen Geräten, wie beispielsweise Injektoren, Safety-Devices oder dergleichen, nimmt jedoch immer weiter zu. Eine Verwendung an bzw. in derartigen medizinischen Geräten gestaltet sich jedoch in den allermeisten Anwendungsfällen als sehr aufwändig, da die Gerätehersteller diese durch Adapter fast immer aufwendig nachrüsten müssen, um insbesondere auch unterschiedliche Glasspritzentypen an ihnen einsetzen zu können. Selbst wenn ein passender Adapter zur Verfügung steht, gestaltet sich eine Montage der Glasspritzen bezüglich dieser medizinischen Geräte oftmals als aufwändig, da es sich im Wesentlichen um Insellösungen handelt.

Beispielsweise zeigt die EP 1 462 134 A1 ein Montagehilfeelement, in dem eine Spritze und insbesondere ein Spritzenkörper arretiert sind. Allerdings sind weder ein Spritzenkonusteil noch ein Flanschteil gezeigt.

Aus der US 3 605 743 A ist ein Spritzensystem bekannt, bei dem eine Spritze in ein Befestigungselement eingeführt ist. Allerdings geht keinerlei Hinweis auf ein Flanschteil und dahingehend hervor, dass ein Referenzpunkt des Montagehilfeelementes immer einen definierten Abstand zu der Nadelspitze der Spritzennadel aufweist.

Die WO 93/10838 A1 zeigt eine Haltevorrichtung für eine Stechvorrichtung, bei der eine Nadel aus dem Körper herausschnellen kann. Allerdings können weder ein Konusteil noch ein Flanschteil entnommen werden.

Die WO 01/07105 A1 zeigt ein Injektionsbauteil umfassend ein Halteelement, in welches eine Spritze eingeführt werden kann, wobei jedoch kein Konusteil gezeigt ist.

Die US 4 518 387 A offenbart ein Injektionselement, bei dem eine Spritze in ein Haltelement eingebracht und in diesem arretiert ist. Dabei weist die Spritze ein vorderes, lediglich abgerundetes Ende auf, welches auf einem Ende des Halteelementes aufliegt. Allerdings ist zunächst überhaupt kein Konusteil an der Spritze gezeigt.

Des Weiteren offenbart die WO 2004/108194 A1 eine Spritze, welche mittels eines Spritzenträgers in einem Gehäuse gelagert ist, wobei der Spritzenträger den zylindrischen Anteil der Spritze umgibt. Gleichzeitig steht er an seinem der Spritzennadel zugewandten Ende mit einem Absatz des Gehäuses in Wechselwirkung. Außerdem ist zwischen dem einen Flansch der Spritze zugewandten Ende des Spritzenträgers und dem Gehäuse eine Feder gespannt. Damit sind also Spritzenträger und Gehäuse zueinander beweglich gelagert.

Es ist Aufgabe der vorliegenden Erfindung, eine konstruktiv einfach bauende Schnittstelle zwischen einer herkömmlichen Glasspritze und einer Glasspritzenbedieneinrichtung bereitzustellen, mittels welcher herkömmliche Glasspritzen einfach und schnell an der Glasspritzenbedieneinrichtung montiert und mit dieser betriebssicherer verwendet werden können.

Im Rahmen der vorliegenden Erfindung wird ein glasspritzenseitiges Montagehilfselement beschrieben, zum Bereitstellen eines Schnittstellenelementes zwischen einer Glasspritze, umfassend vorderseitig ein Spritzenkonusteil mit einer Spritzennadel und umfassend rückseitig ein Flanschteil, und einer Glasspritzenbedieneinrichtung, wobei das Montagehilfselement an der Außenkontur der Glasspritze anordenbar ist, und wobei das Montagehilfselement eine Befestigungseinrichtung aufweist, um das Montagehilfselement bezogen auf die Spritzennadel vor dem Flanschteil an der Glasspritze derart zu befestigen, dass ein Referenzpunkt des Montagehilfselements immer einen definierten Abstand zu der Nadelspitze der Spritzennadel aufweist.

Bei bisher aus dem Stand der Technik bereits bekannten Montagehilfselementen ist deren Befestigungsort an der Glasspritze derart ungünstig am hinteren Flansch der Glasspritze gewählt, dass einerseits die Gefahr einer Spannungsüberhöhung an der Glasspritze besteht, was immer wieder zu einem Glasbruch und damit auch zu einer Zerstörung der Glasspritze führt. Insofern ist es vorteilhaft, dass der Befestigungsort bezogen auf die Spritzennadel erfindungsgemäß vor diesem Flansch positioniert ist. Andererseits ist das Montageelement vorteilhafter Weise stets so an der Glasspritze befestigt, dass ein Referenzpunkt des Montagehilfeelements erfindungsgemäß immer einen definierten Abstand zur Spritzennadelspitze aufweist. Hierdurch wird eine besonders hohe Betriebssicherheit im Umgang mit der Glasspritze erzielt, da der fertigungsbedingte doch recht hoch tolerierte Außenbereich der Glasspritze konstruktiv einfach und kostengünstig gegenüber medizinischen Geräten bzw. Glasspritzenbedieneinrichtungen mit einer definierten Schnittstelle versehen werden kann, wodurch die Position der Spritzennadel innerhalb eines medizinischen Gerätes bzw. einer Glasspritzenbedieneinrichtung außergewöhnlich genau und zuverlässig festgelegt ist. Somit ist auch eine gewünschte Einstechtiefe der Spritzennadel in der Praxis immer genauestens gewährleistet.

Der Begriff "Referenzpunkt" beschreibt im Sinne der Erfindung eine Stelle oder einen Bereich an dem Montagehilfselement, die bzw. der immer im gleichen Abstand bzw. in der gleichen Lage axial wie auch radial zu der Spritzennadelspitze angeordnet ist. Axial bezieht sich hierbei auf die Längserstreckung der Glasspritze, radial quer hierzu. Im einfachsten Fall ist der Referenzpunkt durch eine Kante des Montagehilfselements formuliert.

Das Montagehilfselement ist im Sinne der Erfindung dauerhaft an der Glasspritze angeordnet. Hierzu können insbesondere die weiter unten noch beschriebenen Befestigungsverfahren vorteilhaft eingesetzt werden.

Vorteilhafter Weise ist ein Befestigungsort der Befestigungseinrichtung mehr als 5 mm, vorzugsweise mehr als 10 mm, von dem Flansch der Glasspritze beabstandet angeordnet, sodass insbesondere die Gefahr eines Glasbruchs aufgrund einer kritischen Spannungsüberhöhung weiter reduziert werden kann.

Insgesamt betrachtet, bietet das vorliegende Montagehilfselement eine außerordentlich betriebssichere Halterung der Glasspritze an zusätzlichen Einrichtungen, insbesondere an Glasspritzenbedieneinrichtungen, wie etwa Injektoren, Safety-Devices, Needle-Shields oder dergleichen.

Um das vorliegende die Glasspritze umfassende Montagehilfselement definiert und damit problemlos in derartige Glasspritzenbedieneinrichtungen einsetzen und daran betreiben zu können, ist es vorgesehen, dass das Montagehilfselement eine Halteeinrichtung zum Halten an der Glasspritzenbedieneinrichtung aufweist.

Erfindungsgemäß ist die Halteeinrichtung außen umfangsseitig an dem Montagehilfselement angeordnet. Es kann jedoch auch kumulativ kopfseitig an dem Montagehilfselement platziert sein. Es versteht sich, dass die Halteeinrichtung gerätespezifisch ausgestaltet sein kann. Vorzugsweise ist die Halteeinrichtung standardisiert ausgebildet, sodass deren Herstellung und Anwendung weiter vereinfacht werden kann. Erfindungsgemäß weist die Halteeinrichtung eine spezifische Außenstruktur in Gestalt einer Nut-Feder-Konstruktion auf, wobei diesbezügliche Nuten bzw. Federn in Umfangsrichtung und/oder in Längsrichtung des Montagehilfselements verlaufend angeordnet sind. Es versteht sich, dass erfindungsgemäß an der jeweiligen Glasspritzenbedieneinrichtung eine hiermit wechselwirkende Gegenstruktur vorhanden ist.

Jedenfalls ist es vorteilhaft, wenn das Montagehilfselement insgesamt eine Außengeometrie bereitstellt, welche in Bezug auf deren Maße und insbesondere auf deren Abstand zur Nadelspitze, aber auch zum Flansch der Glasspritze, hin exakt definiert ist.

Es ist erfindungsgemäß gemäß einer ersten Ausführungsvariante vorgesehen, dass die Befestigungseinrichtung einen Aufnahmebereich zur Aufnahme des Spritzenkonusteils aufweist, um das Montagehilfselement an dem Spritzenkonusteil und somit an der Glasspritze zu befestigen.

Der Spritzenkonusteil ist aufgrund seines relativ hohen Anteils an Glasmaterial besonders stabil und damit weniger bruchgefährdet. Da dort auch die Spritzennadel eingebettet angeordnet ist, befindet sich der Spritzenkonusteil in unmittelbarer Nähe zur Spritzennadel. Beides bedingt, dass der Spritzenkonusteil als Befestigungsort für das vorliegende Montagehilfselement im Sinne der Erfindung besonders gut geeignet ist.

Insofern bilden die Merkmale hinsichtlich einer Befestigung des Montagehilfselements an dem Spritzenkonusteil bisher bekannte Adapter auch ohne die übrigen Merkmale der Erfindung vorteilhaft weiter und sind demnach auch unabhängig von diesen übrigen Merkmalen besonders vorteilhaft.

Weist die Befestigungseinrichtung eine umlaufende Nut zum Anordnen einer Umfangserhöhung des Spritzenkonusteils auf, kann sogleich eine hervorragende Abdichtung zwischen dem Montagehilfselement und dem Spritzenkonusteil erreicht werden. Diese Nut kann sich auch erst beim Anspritzen oder Anschweißen des Montagehilfselements an den Spritzenkonusteil durch die Umfangserhöhung ausgestalten.

Alternativ zur ersten Ausführungsvariante ist erfindungsgemäß gemäß einer zweiten Ausführungsvariante vorgesehen, dass die Befestigungseinrichtung einen Aufnahmebereich zur Aufnahme eines Schulterbereichs der Glasspritze aufweist, um das Montagehilfselement an dem Schulterbereich und somit an der Glasspritze zu befestigen.

Auch der Schulterbereich der Glasspritze stellt einen relativ stabilen und bruchsicheren Befestigungsort an der Glasspritze zur Verfügung, an welchem das vorliegende Montagehilfselement äußerst präzise befestigt werden kann. Deshalb ist an dem Schulterbereich die Gefahr eines Glasbruchs der Glasspritze gegenüber herkömmlichen Befestigungsorten ebenfalls stark reduziert.

Es ist auch möglich, das Montagehilfselement kumulativ an diesem Schulterbereich der Glasspritze und damit vorteilhaft an der Glasspritze anzubringen.

An dieser Stelle sei auch darauf hingewiesen, dass die Merkmale hinsichtlich einer Befestigung des Montagehilfselements an dem Schulterbereich der Glasspritze bisher bekannte Adapter auch ohne die übrigen Merkmale der Erfindung vorteilhaft weiterbilden. Insofern wird die Aufgabe der Erfindung allein schon von diesen Merkmalen bezüglich der Befestigung gelöst.

Hinsichtlich einer dritten Ausführungsvariante ist es vorgesehen, dass die Befestigungseinrichtung einen Aufnahmebereich zur Aufnahme eines Zylinderbereichs der Glasspritze aufweist, wobei der Zylinderbereich eine Längserstreckung größer als dessen Durchmesser aufweist, um das Montagehilfselement an dem Zylinderbereich und somit an der Glasspritze zu befestigen.

Vorteilhafter Weise kann mittels eines derartigen Zylinderbereichs ein Befestigungsort an der Glasspritze bereitgestellt werden, der eine größtmögliche Anlagefläche formuliert, sodass Kräfte zwischen dem Montagehilfselement und der Glasspritze besonders großflächig übertragen werden können. Hierdurch ist eine Glasbruchgefahr an der Glasspritze kumulativ oder alternativ gut reduziert. Ebenso kann auch hier eine definierte Lageposition des Montagehilfselements in Bezug auf die Spritzennadel vorteilhaft erreicht werden.

Somit bilden die Merkmale hinsichtlich einer Befestigung des Montagehilfselements an dem Zylinderbereich bisher bekannte Adapter ebenfalls auch ohne die übrigen Merkmale der Erfindung weiter und sind demnach auch unabhängig von diesen übrigen Merkmalen besonders vorteilhaft. Allerdings ist die dritte Ausführungsvariante, wonach die Befestigungseinrichtung den Aufnahmebereich zur Aufnahme des Zylinderbereichs der Glasspritze aufweist, vorliegend lediglich beispielhaft beschrieben.

Vorzugsweise liegt der Zylinderbereich zwischen dem Schulterbereich und dem Flanschteil der Glasspritze, sodass das Montagehilfselement auch in diesem Fall wieder vor dem Flansch angeordnet ist. Insofern beschreibt der Begriff "Zylinderbereich" einen im Wesentlichen zylindrisch ausgestalteten Abschnitt der Glasspritze, der zwischen dem Schulterbereich und dem Flanschteil liegt.

In diesem Zusammenhang sei noch erwähnt, dass der Begriff "Schulterbereich" einen Abschnitt der Glasspritze beschreibt, in welchem sich die Glasspritze ausgehend von dem Spritzenkonusteil radial aufweitet und in den Zylinderbereich der Glasspritze übergeht.

Im Rahmen der vorliegenden Erfindung wird weiter ein glasspritzenseitiges Montagehilfselement beschrieben, zum Bereitstellen eines Schnittstellenelementes zwischen einer Glasspritze und einer Glasspritzenbedieneinrichtung, wobei das Montagehilfselement an der Außenkontur der Glasspritze anordenbar ist, wobei das Montagehilfselement einen der Glasspritze zugewandten Innenkontaktbereich und einen der Glasspritze abgewandten Glasspritzenbedieneinrichtungskontaktbereich umfasst, und wobei der Innenkontaktbereich weicher ausgestaltet ist als der Glasspritzenbedieneinrichtungskontaktbereich.

Dadurch, dass der Innenkontaktbereich des Montagehilfselements mit weicheren Materialeigenschaften ausgestattet ist, kann sich das Montagehilfselement wesentlich inniger an die Glasspritze anschmiegen, wodurch eine Wechselwirkung zwischen diesen beiden Bauteilen außerordentlich intensiv gestaltet werden kann. Insofern kann allein hierdurch schon eine erhebliche Glasbruchgefahrreduzierung an der Glasspritze erzielt werden.

In diesem Zusammenhang wird die Aufgabe der Erfindung auch von einem Verfahren zum Herstellen eines Montagehilfselements zum Bereitstellen eines Schnittstellenelementes zwischen einer Glasspritze und einer Glasspritzenbedieneinrichtung gelöst, bei welchem das Montagehilfselement mindestens zweilagig hergestellt wird, wobei es an einer der Glasspritze zugewandten Seite weicher ausgestaltet wird als an einer der Glasspritze abgewandten Seiten, an welcher die Glasspritzenbedieneinrichtung angeordnet wird.

Hierdurch kann im Sinne der Erfindung ein besonders vorteilhaftes Mehrkomponentenbauteil verfahrenstechnisch einfach zur Verfügung gestellt werden.

Des Weiteren kann das Montagehilfselement hinsichtlich eines Patienten- und Arbeitsschutzes besonders günstig weiterentwickelt werden, wenn das Montagehilfselement ein Schutzkappenteil zum Abdecken der Spritzennadel umfasst, welches mittels einer Sollbruchstelle an dem Montagehilfselement angeordnet ist. Das Schutzkappenteil kann hierbei ein multifunktionales Needle-Shield formulieren, welches einerseits die Funktion der Sicherung und Abschirmung einer Nadelspritze gewährleisten kann. Andererseits kann sich an seinem hinteren proximalen Ende das Montagehilfselement anschließen, welches nach dem Entfernen des Schutzkappenteils an der Glasspritze verbleibt. Ein besonders leichtes Ablösen bzw. Abbrechen des Schutzkappenteils von dem Montagehilfselement kann durch die Sollbrauchstelle sichergestellt werden.

Vorteilhafter Weise besteht das Montagehilfselement aus Kunststoff oder einem Komposit hiervon. Andere Ausführungsvarianten können jedoch auch vorsehen, dass das Montagehilfselement aus einem Metall oder einer Metalllegierung hergestellt ist. Es versteht sich, dass auch andere gut urformbare und umformbare Materialien zum Einsatz kommen können.

Die Aufgabe der Erfindung wird also gelöst von einer Anordnung aus einer Glasspritze und aus einem Montagehilfselement, zum Bereitstellen eines Schnittstellenelementes zwischen der Glasspritze, welche vorderseitig ein Spritzenkonusteil mit einer Spritzennadel und rückseitig ein Flanschteil umfasst, und einer Glasspritzenbedieneinrichtung, wobei das Montagehilfselement bezogen auf die Spritzennadel vor dem Flanschteil an der Glasspritze derart befestigt ist, dass ein Referenzpunkt des Montagehilfselements immer einen definierten Abstand und die gleiche Lage axial wie auch radial zu der Spritzennadelspitze aufweist, wobei das Montagehilfselement an einer Außenkontur der Glasspritze angeordnet ist, wobei das Montagehilfselement an der Glasspritze angespritzt, angeklebt oder angeschweißt angeordnet ist. Erfindungsgemäß weist das Montagehilfselement eine Befestigungseinrichtung auf, mittels der das Montagehilfselement bei einer Anordnung an der Außenkontur der Glasspritze bezogen auf die Spritzennadel vor dem Flanschteil an der Glasspritze derart befestigt ist, dass stets ein definierter Abstand zwischen einem Referenzpunkt des Montagehilfeelements und der Nadelspitze der Spritzennadel ausgebildet ist. Erfindungsgemäß weist die Befestigungseinrichtung einen Aufnahmebereich zur Aufnahme des Spritzenkonusteils auf, mittels welcher das Montagehilfselement an dem Spritzenkonusteil und somit an der Glasspritze befestigt ist, oder die Befestigungseinrichtung weist einen Aufnahmebereich zur Aufnahme eines Schulterbereichs der Glasspritze auf, wobei das Montagehilfselement an dem Schulterbereich und somit an der Glasspritze befestigt ist. Erfindungsgemäß weist das Montagehilfselement eine außen umfangsseitig an dem Montagehilfselement angeordnete Halteeinrichtung auf, mittels welcher das Montagehilfselement an der Glasspritzenbedieneinrichtung gehalten ist, wobei die Halteeinrichtung eine spezifische Außenstruktur in Gestalt einer Nut-Feder-Konstruktion aufweist, wobei diesbezügliche Nuten bzw. Federn in Umfangsrichtung und/oder in Längsrichtung des Montagehilfselements verlaufend angeordnet sind und wobei an der Glasspritzenbedieneinrichtung eine mit der Halteeinrichtung wechselwirkende Gegenstruktur ausgestaltet ist.

Ein hier lediglich beispielhaft beschriebenes Verfahren zum Befestigen eines Montagehilfselements an einer Glasspritze sieht vor, dass eine Befestigungseinrichtung des Montagehilfselements bezogen auf eine Spritzennadel der Glasspritze vor einem Flanschteil der Glasspritze außen an der Glasspritze angebracht wird, indem die Befestigungseinrichtung an die Außenkontur der Glasspritze angespritzt wird.

Durch dieses Anspritzen der Befestigungseinrichtung an die Außenkontur der Glasspritze kann einerseits die Fügegeometrie des Montagehilfselements bzw. der Befestigungseinrichtung exakt an die hochtolerierte Außengestalt der Glasspritze angepasst werden. Andererseits kann herstellungstechnisch einfach nahezu jede erdenkliche und praktikable Halteeinrichtungsgeometrie zum Halten in der Glasspritzenbedieneinrichtung bereitgestellt werden.

Es kann jedoch sein, dass beim Umspritzen der Glasspritze eine bestimmte Abdichtung eines Einlegeteils erforderlich ist, welches zudem einem Innendruck am Spritzwerkzeug standhält, jedoch die Gefahr eines Glasbruchs an der Glasspritze reduzieren bzw. zur Gänze ausschließen kann. Vorteilhafter Weise kann dieses Einlegeteil sogleich den vorstehend beschriebenen weicheren Innenkontaktbereich des Montagelements bilden.

Eine weitere hier lediglich beispielhaft beschriebene Verfahrensvariante sieht vor, dass eine Befestigungseinrichtung des Montagehilfselements bezogen auf eine Spritzennadel der Glasspritze vor einem Flanschteil der Glasspritze außen an der Glasspritze angebracht wird, indem die Befestigungseinrichtung an die Außenkontur der Glasspritze angeklebt wird.

Hierbei können jegliche aus medizinischer Sicht unkritische Kleber verwendet werden. Insbesondere auch UV-härtende Kleber können vorteilhaft zum Einsatz kommen, welche schnell aushärtend eine innige Verbindung direkt an der eigentlichen Kontaktfläche zwischen dem Montagehilfselement und der Glasspritze bewirken können.

Besonders vorteilhaft ist es jedoch, wenn eine Befestigung mittels eines Klebeetiketts oder dergleichen bewerkstelligt wird. Die Verwendung derartiger Klebeetikette ist aus dem Stand der Technik in anderer Funktion, wie beispielsweise hinsichtlich angeklebter Spritznadelabdeckeinrichtungen, bekannt und hat sich dort bereits gut bewährt. Insbesondere mittels des vorliegend bevorzugten Klebeetiketts kann die Fügegeometrie des Montagehilfselements bzw. der Befestigungseinrichtung gut an die hochtolerierte Außengestalt der Glasspritze angepasst werden. Aufgrund der sehr einfachen Verfahrensweise wird eine Befestigung mittels eines Klebeetiketts vorliegend bevorzugt vor den übrigen Befestigungsverfahren angewendet.

Eine alternative und nicht minder vorteilhafte Verfahrensvariante sieht vor, dass eine Befestigungseinrichtung des Montagehilfselements bezogen auf eine Spritzennadel der Glasspritze vor einem Flanschteil der Glasspritze außen an der Glasspritze angebracht wird, indem die Befestigungseinrichtung an die Außenkontur der Glasspritze, und zwar an das Spritzenkonusteil und an die Umfangserhöhung, angeschweißt wird.

Das Montagehilfselement kann vorteilhaft auch beispielsweise mittels eines Ultraschall- oder Laserschweißverfahrens an die Glasspritze befestigt werden. Auch hierbei kann durch Anschmelzen von Montagehilfselementmaterial die Fügegeometrie des Montagehilfselements bzw. der Befestigungseinrichtung sehr gut an die hochtolerierte Außengestalt der Glasspritze angepasst werden.

Zusammenfassend können die vorliegend insgesamt erzielbaren Vorteile der Erfindung auch für die erfindungsgemäße Anordnung wie folgt festgehalten werden. Erstens wird die Gefahr eines Glasbruchs an der Glasspritze vermieden, da eine Befestigung des Montagehilfselements nicht über den Flansch der Glasspritze erfolgt. Zweitens wird eine wesentlich bessere Definition der Einstechtiefe der Spritzennadel durch Toleranzumgehung mittels des Montagehilfselements erzielt. Drittens entfallen aufwendige Einzelschnittstellenlösungen an den medizinischen Geräten, gegebenenfalls kann hierdurch ein einheitlicher Industriestandard geschaffen werden. Viertens werden Herstellungskosten für ein Gesamtsystem Glasspritze und Glasspritzenbedieneinrichtung erheblich reduziert. Fünftens kann eine vorteilhafte Kombinationslösung Montagehilfselement mit Schutzkappenteil für eine Needle-Shield-Funktion angeboten werden.

Vorteilhafter Weise umfasst die Anordnung ein Montagehilfselement nach einem der hier beschriebenen Merkmale.

Weitere Vorteile, Ziele und Eigenschaften vorliegender Erfindung werden anhand anliegender Zeichnung und nachfolgender Beschreibung erläutert, in welchen beispielhaft einige glasspritzenseitige Montagehilfselemente in Anordnung mit einer Glasspritze dargestellt und beschrieben sind. In der Zeichnung zeigen:
- Figur 1A: schematisch eine Explosionsansicht einer Anordnung aus einem Montagehilfselement, einer Glasspritze und einer Glasspritzenbedieneinrichtung;
- Figur 1B: schematisch eine weitere Ansicht der Anordnung aus der Figur 1A in einem zusammengesetzten Zustand;
- Figur 1C: schematisch eine längsgeschnittene Ansicht der Anordnung aus den Figuren 1A und 1B;
- Figur 1D: schematisch eine längsgeschnittene Detailansicht der Anordnung aus den Figuren 1A bis 1C hinsichtlich eines Spritzenkonusteils der Glasspritze;
- Figur 2A: schematisch eine Explosionsansicht einer weiteren Anordnung aus einer Glasspritze und einem daran zylinderbereichsseitig befestigten Montagehilfselement;
- Figur 2B: schematisch eine längsgeschnittene Detailansicht der Anordnung aus der Figur 2A in einem zusammengesetzten Zustand;
- Figur 2C: schematisch eine perspektivische Ansicht des Montagehilfselements aus den Figuren 2A und 2B;
- Figur 3: schematisch eine längsgeschnittene Ansicht einer ähnlichen Anordnung aus einer Glasspritze und einem daran zylinderbereichsseitig befestigten ähnlichen Montagehilfselement wie aus den Figuren 2A bis 2C;
- Figur 4A: schematisch eine Explosionsansicht einer anderen Anordnung aus einer Glasspritze und einem daran schulterbereichsseitig befestigten Montagehilfselement;
- Figur 4B: schematisch eine perspektivische Detailansicht der Anordnung aus der Figur 4A in einem zusammengesetzten Zustand;
- Figur 5A: schematisch eine weitere Explosionsansicht einer alternativen Anordnung aus einer Glasspritze und einem daran spritzenkonusteilseitig befestigten Montagehilfselement;
- Figur 5B: schematisch eine längsgeschnittene Detailansicht der alternativen Anordnung aus der Figur 5A in einem zusammengesetzten Zustand;
- Figur 5C: schematisch eine perspektivische Ansicht des noch nicht befestigten Montagehilfselements aus den Figuren 5A und 5B mit einem aufgesetzten Schutzkappenteil;
- Figur 5D: schematisch eine perspektivische Ansicht des befestigten Montagehilfselements aus den Figuren 5A bis 5C mit einem von dem Montagehilfselement abgezogenen Schutzkappenteil;
- Figur 6A: schematisch eine perspektivische Ansicht eines Montagehilfselements mit einem daran über eine Sollbruchstelle angeordneten Schutzkappenteil zum Abdecken einer Spritzennadel; und
- Figur 6B: schematisch eine längsgeschnittene Ansicht des Montagehilfselements aus der Figur 6A.

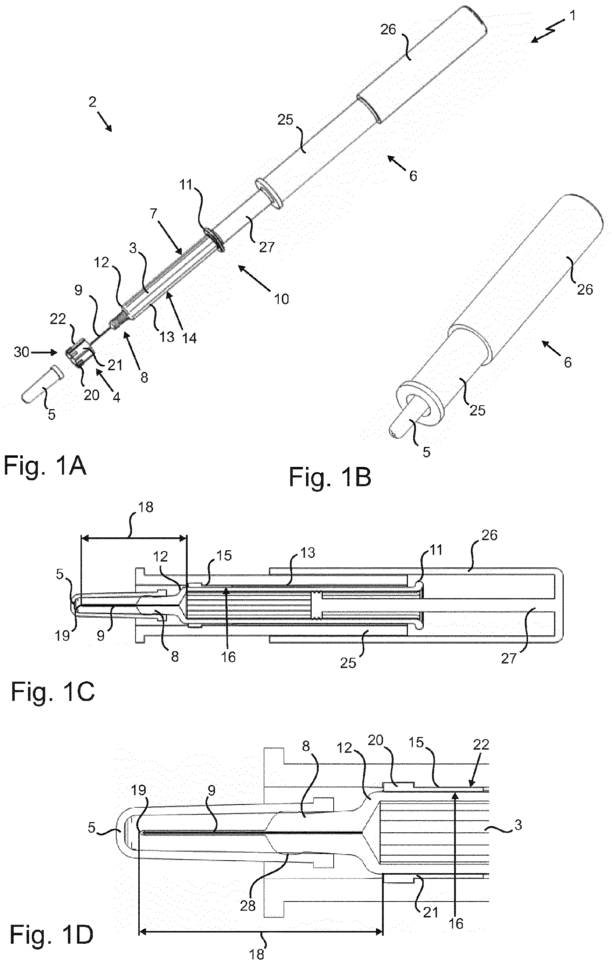

Die Figuren 1A bis 1D, 2A bis 2C und 3 zeigen dabei wesentliche Elemente der vorliegenden Erfindung, sind jedoch in Bezug auf die Befestigung zwischen Montagehilfselement und Außenseite der Glasspritze als lediglich beispielhafte Ausführungen anzusehen.

Das in den Figuren 1A bis 1D dargestellte erste Ausführungsbeispiel 1 zeigt eine erste Anordnung 2 aus einer handelsüblichen Glasspritze 3, einem Montagehilfselement 4, einem Schutzkappenteil 5 und einer Glasspritzenbedieneinrichtung 6.

Die Glasspritze 3 umfasst einen Grundkörper 7 mit einem vorderseitigen Spritzenkonusteil 8, mittels welchem eine Spritzennadel 9 an dem Grundkörper 7 der Glasspritze 3 eingebettet angeordnet ist. Der Grundkörper 7 weist des Weiteren an dem hinteren Ende 10 der Glasspritze 3 ein Flanschteil 11 auf. Zwischen dem Spritzenkonusteil 8 und dem Flanschteil 11 liegen ein Schulterbereich 12 und ein Zylinderbereich 13. Insgesamt gestalten der Spritzenkonusteil 8, der Schulterbereich 12, der Zylinderbereich 13 und der Flanschteil 11 die Außenkontur 14 der Glasspritze 3 aus.

Das Montagehilfselement 4 weist eine Befestigungseinrichtung 15 (siehe insbesondere Figuren 1C und 1D) mit einem Innenkontaktbereich 16 auf, welcher der Glasspritze 3 zugewandt ist. Mittels der Befestigungseinrichtung 15 ist das Montagehilfselement 4 an der Glasspritze 3 befestigt.

Das Montagehilfselement 4 weist des Weiteren in Umfangsrichtung mehrere Referenzpunkte (hier nicht beziffert) auf, mittels welcher immer ein definierter Abstand 18 zu der Nadelspitze 19 der Spritzennadel 9 gewährleistet werden kann.

Die Referenzpunkte können beispielsweise mittels Federelemente 20 einer Halteeinrichtung 21 bereitgestellt werden, mittels welcher die Glasspritzenbedieneinrichtung 6 an dem Montagehilfeelement 4 gehaltert werden kann. Die Federelemente 20 sind hierbei in einem Glasspritzenbedieneinrichtungskontaktbereich 22 an der Außenseite des Montagehilfeelements 4 vorgesehen, wobei die Federelemente 20 mit entsprechenden Nuten (hier nicht beziffert) der Glasspritzenbedieneinrichtung 6 in Art einer Feder-Nut-Verbindungseinrichtung wechselwirken.

Die Glasspritzenbedieneinrichtung 6 besteht zumindest in diesem ersten Ausführungsbeispiel 1 aus einem Halteteil 25, an welchem ein Schubteil 26 mit einem Kolbenteil 27 translatorisch beweglich gelagert ist.

Am Spritzenkonusteil 8 ist noch eine Umfangserhöhung 28 vorgesehen. An dieser kann in diesem ersten Ausführungsbeispiel 1 das Schutzkappenteil 5 verliersicher gehalten werden.

In den Figuren 1B bis 1D ist die Anordnung 2 in einem Lieferzustand gezeigt, in welchem die Glasspritzenbedieneinrichtung 6 noch nicht betätigt wurde und das Schutzkappenteil 5 noch auf dem Spritzenkonusteil 8 aufgesteckt ist.

Das Montagehilfselement 4 stellt ein besonders vorteilhaftes Schnittstellenelement 30 zwischen der Glasspritze 3 und der Glasspritzenbedieneinrichtung 6 dar.

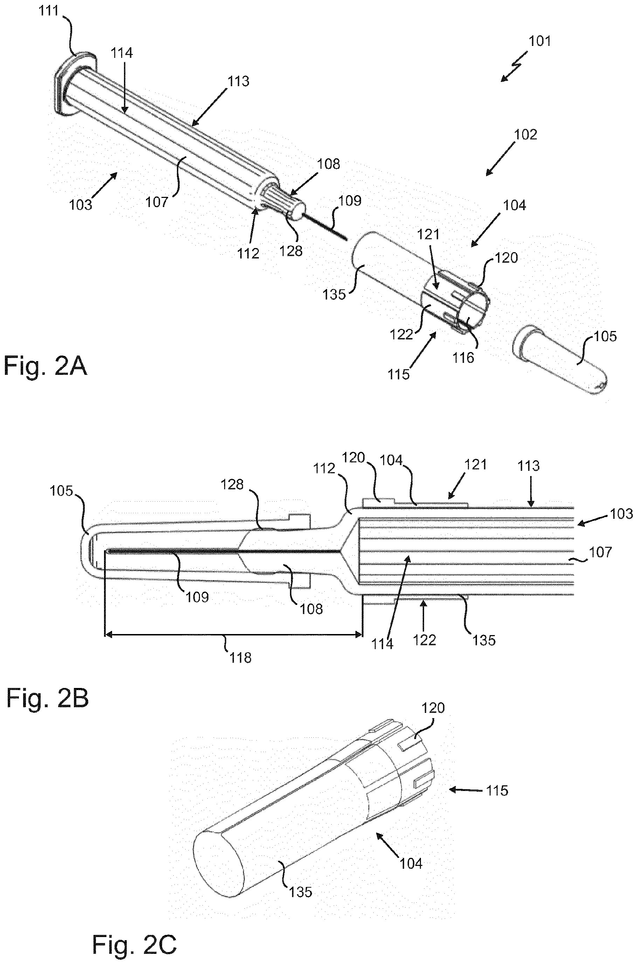

Das in den Figuren 2A bis 2C dargestellte zweite Ausführungsbeispiel 101 zeigt eine Anordnung 102 aus einer handelsüblichen Glasspritze 103, einem Montagehilfselement 104 und einem Schutzkappenteil 105, wobei die Glasspritze 103 einen Grundkörper 107 mit einem vorderseitigen Spritzenkonusteil 108 umfasst, mittels welchem eine Spritzennadel 109 an der Glasspritze 103 eingebettet angeordnet ist. Der Grundkörper 107 weist des Weiteren an seinem hinteren Ende 110 ein Flanschteil 111 auf. Zwischen dem Spritzenkonusteil 108 und dem Flanschteil 111 liegen wieder ein Schulterbereich 112 und ein Zylinderbereich 113 der Glasspritze 103.

Das Montagehilfselement 104 weist eine Befestigungseinrichtung 115 mit einem Innenkontaktbereich 116 auf, welcher der Glasspritze 103 zugewandt ist. Insofern ist das Montagehilfselement 104 mittels der Befestigungseinrichtung 115 an der Glasspritze 103 befestigt (siehe Figur 2B). Darüber hinaus weist es in Umfangsrichtung mehrere Referenzpunkte in Gestalt von Federelementen 120 auf, mittels welcher immer ein definierter Abstand 118 zu der Nadelspitze 119 der Spritzennadel 109 gewährleistet ist.

Die Federelemente 120 werden von einer Halteeinrichtung 121 bereitgestellt, mittels welcher eine Glasspritzenbedieneinrichtung (siehe Figuren 1) an dem Montagehilfselement 104 gehaltert ist. Die Federelemente 120 sind in einem Glasspritzenbedieneinrichtungskontaktbereich 122 an der Außenseite des Montagehilfselements 104 vorgesehen.

Wie unschwer zu erkennen ist, ist das Montagehilfselement 104 auch hier in dem Zylinderbereich 113 der Glasspritze 103 befestigt, wobei das Montagehilfselement 104 wieder mit einem teilweise beidseitig klebenden Klebeetikett 135 an der Glasspritze 103 befestigt ist. Hierzu ist das teilweise beidseitig klebende Klebeetikett 135 einfach auf die Außenkontur 114 der Glasspritze 103 aufgeklebt.

Insofern stellt das Montagehilfselement 104 auch hier wieder ein konstruktiv vorteilhaftes Schnittstellenelement 130 für die Glasspritze 103 bereit.

Am Spritzenkonusteil 108 ist wieder eine Umfangserhöhung 128 vorgesehen. An dieser kann das Schutzkappenteil 105 verliersicher gehalten werden.

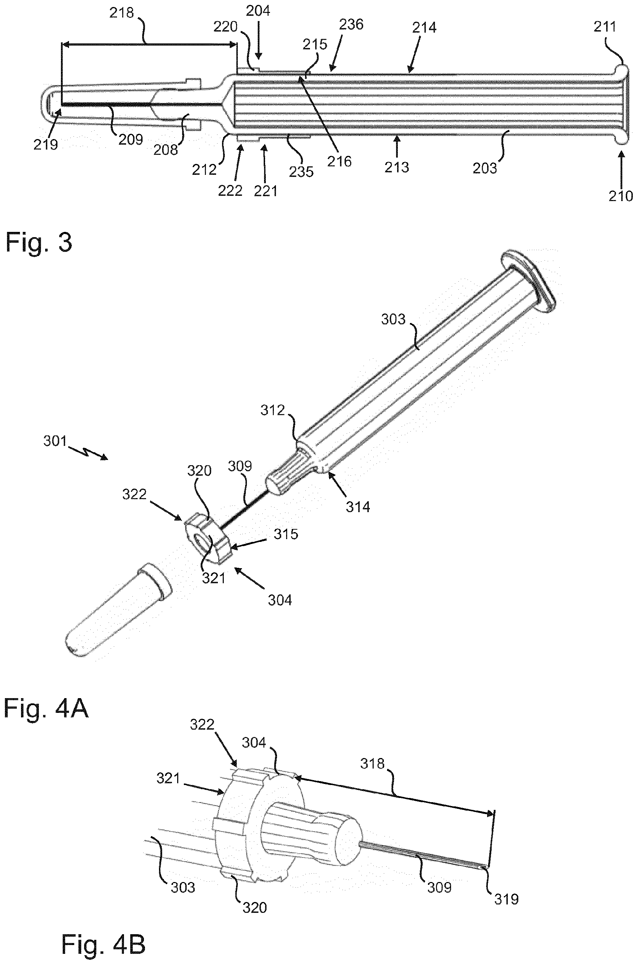

Das weitere in der Figur 3 gezeigte Montagehilfselement 204 entspricht vom Aufbau her im Wesentlichen dem Montagehilfselement 104 aus den Figuren 1 und es ist ebenfalls mittels eines einseitig klebenden Klebeetiketts 235 an einer Glasspritze 203 befestigt; und zwar ebenfalls in einem Zylinderbereich 213 der Glasspritze 203.

Jedoch flacht sich das Montagehilfselement 204 im hinteren Bereich 236 stärker ab, sodass dort ein noch flacherer Übergang zur Glasspritze 203 geschaffen ist, als dies bei den vorherigen Ausführungsbeispielen der Fall ist. Gegebenenfalls kann das Montagehilfselement 204 hinsichtlich einer zusätzlichen Befestigungsmöglichkeit vorteilhafter mit einem bis auf die Außenkontur 214 der Glasspritze 203 gewickelten Tapemittel (hier nicht gezeigt) nochmals verbessert an der Glasspritze 203 befestigt werden.

Ansonsten weist das Montagehilfselement 204 eine Befestigungseinrichtung 215 mit einem Innenkontaktbereich 216 auf, um mit dem Klebeetikett 235 großflächig zu verkleben. Außen am Montagehilfselement 204 ist eine Halteeinrichtung 221 zum Halten einer Glasspritzenbedieneinrichtung an einem Glasspritzenbedieneinrichtungskontaktbereich 222 vorgesehen, welcher wiederum Federelemente 220 zum Etablieren einer Nut-Feder-Verbindungseinrichtung zwischen dem Montagehilfselement 204 und der Glasspritzenbedieneinrichtung umfasst.

Die Federelemente 220 definieren wieder Referenzpunkte an dem Montagehilfselement 204, anhand derer ein definierter Abstand 218 zwischen dem Montagehilfselement 204 und einer Nadelspitze 219 bestimmt werden kann.

Die Glasspritze 203 besitzt wieder einen Aufbau mit einem Grundkörper 207, der vorderseitig einen Spritzenkonusteil 208 mit einer Spritzennadel 209, einen daran anschließenden Schulterbereich 212, den darauf folgenden Zylinderbereich 213 und am Ende 210 der Glasspritze 203 ein Flanschteil 211 umfasst.

Bei dem in den Figuren 4A und 4B gezeigten weiteren Ausführungsbeispiel 301 handelt es sich wieder um eine Glasspritze 303 der vorgenannten Bauart, sodass auf eine detaillierte Beschreibung nun verzichtet wird, um auch Wiederholungen zu vermeiden. Jedenfalls weist die Glasspritze 303 einen Schulterbereich 312 auf, an welchem ein Montagehilfselement 304 angespritzt ist. Insofern stellt das Montagehilfselement 304 innenseitig eine Befestigungseinrichtung 315 zur Verfügung, mittels welcher es an dem Schulterbereich 312 und damit problemlos an einer Außenkontur 314 der Glasspritze 303 befestigt werden kann. Auch hierbei kann ein definierter Abstand 318 anhand von Referenzpunkten in Gestalt von Federelementen 320 zwischen dem Montagehilfselement 304 und einer Nadelspritze 319 einer Spritzennadel 318 erzeugt werden. Die Federelemente 320 gehören, wie hinsichtlich der vorstehend erläuterten Ausführungsbeispiele auch, zu einer Feder-Nut-Verbindung zwischen einem Glasspritzenbedieneinrichtungskontaktbereich 322 einer montagehilfselementseitigen Halteeinrichtung 321 und einer hier nicht gezeigten Glasspritzenbedieneinrichtung.

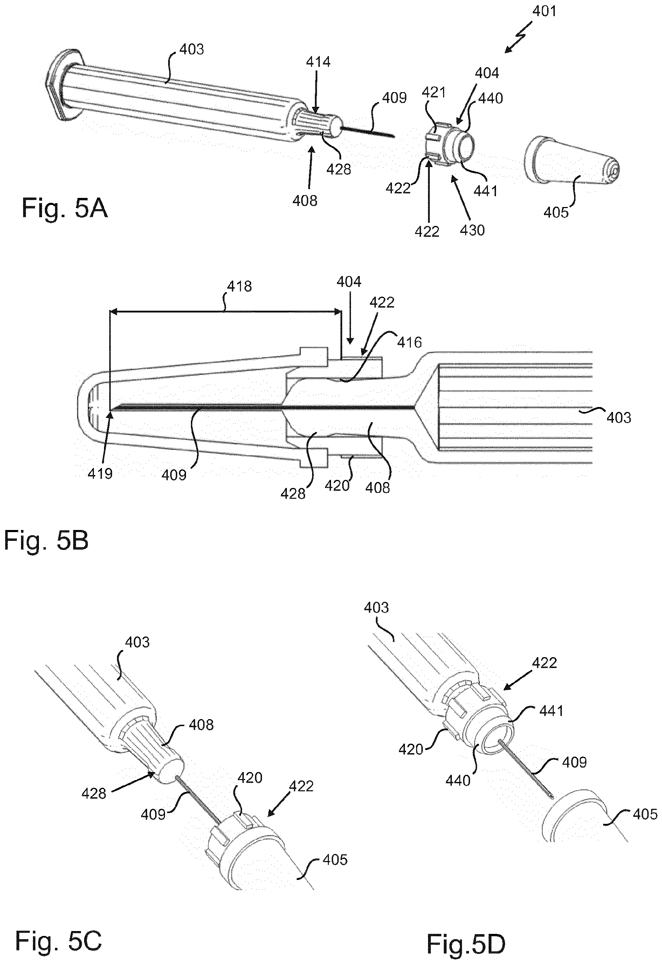

Bei einem nächsten in den Figuren 5A bis 5D gezeigten Ausführungsbeispiel 401 ist ein Montagehilfselement 404 an einem Spritzenkonusteil 408 einer Glasspritze 403 vorteilhaft angeschweißt. Da die Glasspritze 403 auch in diesem nächsten Ausführungsbeispiel 401 identisch mit den zuvor beschriebenen Glasspritzen ist, werden hier nur die wesentlichen Unterschiede zu den vorstehenden Ausführungsbeispielen beschrieben.

Wie insbesondere gemäß der Darstellung nach der Figur 5B gut ersichtlich ist, sitzt das Montagehilfselement 404 mit seiner Befestigungseinrichtung 415 auf dem Spritzenkonusteil 408 sowie einer dort ausgebildeten Umfangserhöhung 428 auf, um das Montagehilfselement 404 an dem Spritzenkonusteil 408 und somit an einer Außenkontur 414 der Glasspritze 403 vorteilhaft zu befestigen.

Hierzu ist ein Innenkontaktbereich 416 mittels eines Laserschweißverfahrens angeschmolzen worden, sodass das Montagehilfselement 404 vorteilhaft besonders innig mit dem Spritzenkonusteil 408 verbunden ist.

Das Montagehilfselement 404 hat außen wieder eine Halteeinrichtung 421 zum Halten an einer hier nicht gezeigten Glasspritzenbedieneinrichtung. Das Montagehilfselement 404 bzw. die Halteeinrichtung 421 umfasst einen Glasspritzenbedieneinrichtungskontaktbereich 422 mit Federelementen 420 zum Etablieren einer Feder-Nut-Verbindungseinrichtung.

Des Weiteren liegt zwischen dem Montagehilfselement 404 und der Nadelspitze 419 einer Spritzennadel 409 der Glasspritze 403 wieder ein definierter Abstand 418.

Insofern stellt das Montagehilfselement 404 ein weiteres vorteilhaftes Schnittstellenelement 430 an der Glasspritze 403 dar.

Im Unterschied zu den vorstehend erläuterten Ausführungsbeispielen ist ein Schutzkappenteil 405 nicht an dem Spritzenkonusteil 408 angeordnet, da dort dass Montagehilfselement 404 sitzt. Aus diesem Grund zeichnet sich das Montagehilfselement 404 durch eine Aufsteckeinrichtung 440 zum Ausstecken des Schutzkappenteils 405 auf das Montagehilfselement 404 aus. Die Aufsteckeinrichtung 440 weist einen vorderen konzentrisch umlaufenden Wulst 441 eines Hinterschneidungselements auf, hinter welchem das Schutzkappenteil 405 einrasten kann.

Gemäß der Darstellung nach der Figur 5C ist das mit dem Schutzkappenteil 405 ausgestattete Montagehilfselement 404 noch nicht mit dem Laserschweißverfahren auf das Spritzenkonusteil 408 der Glasspritze 403 aufgeschmolzen.

Gemäß der Darstellung nach der Figur 5D ist das Schutzkappenteil 405 von dem nun fertig montierten Montagehilfselement 404 abgezogen und die Glasspritze 403 ist mit dem Montagehilfselement 404 einsatzbereit.

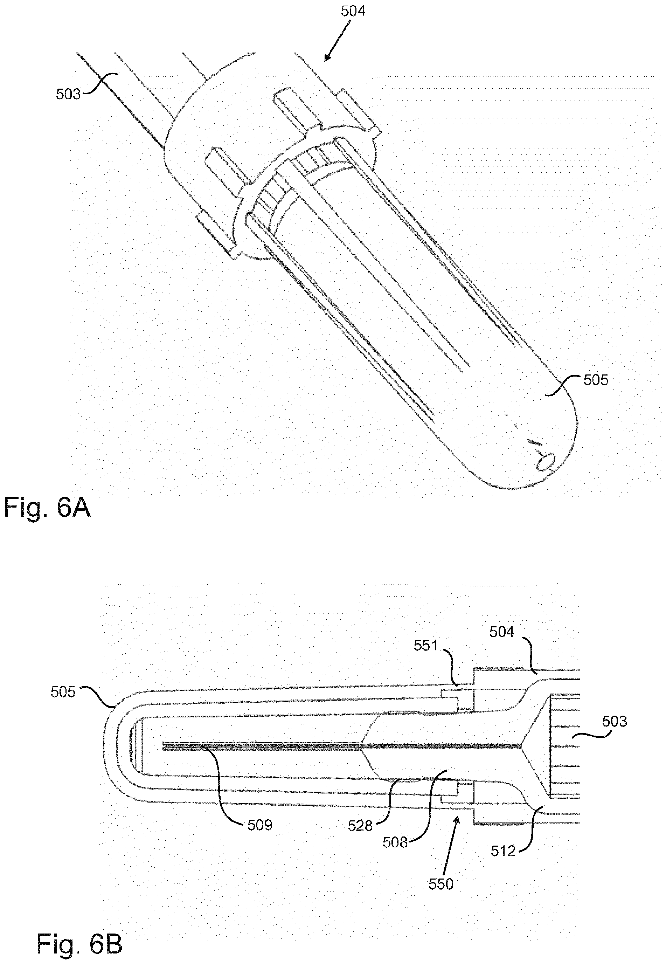

Bei dem in den Figuren 6A und 6B gezeigten Montagehilfselement 504, welches an einem Schulterbereich 512 einer Glasspritze 503 angespritzt ist, ist ein Schutzkappenteil 505 zum Abdecken einer Spritzennadel 509 mittels eines dünnen umlaufenden Verbindungsstegs 550 an dem Montagehilfselement 504 befestigt. Der dünne Verbindungssteg 550 stellt eine Sollbruchstelle 551 dar, sodass das Schutzkappenteil 505 einerseits verliersicher vormontiert bereitgestellt werden kann. Andererseits kann das herstellungsseitig verliergesicherte Schutzkappenteil 505 aufgrund der Sollbruchstelle 551 einfach von dem Montagehilfselement 504 abgetrennt werden. Zusätzlich ist das Schutzkappenteil 505 auf einer Umfangserhöhung 528 eines Spritzenkonusteils 508 der Glasspritze 503 gehaltert, sodass es auch nach einem durchtrennten Verbindungssteg 550 wieder an der Glasspritze 503 befestigt werden kann.

Es versteht sich, dass es sich bei den vorstehend erläuterten Ausführungsbeispielen lediglich um erste Ausgestaltungen von erfindungsgemäßen glasspritzenseitigen Montagehilfselementen handelt. Insofern beschränkt sich die Ausgestaltung der Erfindung nicht auf diese Ausführungsbeispiele.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: erstes Ausführungsbeispiel
- 2: erste Anordnung
- 3: Glasspritze
- 4: Montagehilfselement
- 5: Schutzkappenteil
- 6: Glasspritzenbedieneinrichtung
- 7: Grundkörper
- 8: Spritzenkonusteil
- 9: Spritzennadel
- 10: hinteres Ende
- 11: Flanschteil
- 12: Schulterbereich
- 13: Zylinderbereich
- 14: Außenkontur
- 15: Befestigungseinrichtung
- 16: Innenkontaktbereich
- 18: Abstand
- 19: Nadelspitze
- 20: Federelemente
- 21: Halteeinrichtung
- 22: Glasspritzenbedieneinrichtungskontaktbereich
- 25: Halteteil
- 26: Schubteil
- 27: Kolbenteil
- 28: Umfangserhöhung
- 30: Schnittstellenelement

- 101: zweites Ausführungsbeispiel
- 102: Anordnung
- 103: Glasspritze
- 104: Montagehilfselement
- 105: Schutzkappenteil
- 107: Grundkörper
- 108: Spritzenkonusteil
- 109: Spritzennadel
- 111: Flanschteil
- 112: Schulterbereich
- 113: Zylinderbereich
- 114: Außenkontur
- 115: Befestigungseinrichtung
- 116: Innenkontaktbereich
- 118: Abstand
- 119: Nadelspitze
- 120: Federelemente
- 121: Halteeinrichtung
- 122: Glasspritzenbedieneinrichtungskontaktbereich
- 128: Umfangserhöhung
- 130: Schnittstellenelement
- 135: Klebeetikett

- 203: Glasspritze
- 204: Montagehilfselement
- 207: Grundkörper
- 208: Spritzenkonusteil
- 209: Spritzennadel
- 210: hinteres Ende
- 211: Flanschteil
- 212: Schulterbereich
- 213: Zylinderbereich
- 214: Außenkontur
- 215: Befestigungseinrichtung
- 216: Innenkontaktbereich
- 218: Abstand
- 219: Nadelspitze
- 220: Federelemente
- 221: Halteeinrichtung
- 222: Glasspritzenbedieneinrichtungskontaktbereich
- 236: hinterer Bereich

- 301: weiteres Ausführungsbeispiel
- 303: Glasspritze
- 304: Montagehilfselement
- 309: Spritzennadel
- 312: Schulterbereich
- 314: Außenkontur
- 315: Befestigungseinrichtung
- 318: Abstand
- 319: Nadelspitze
- 320: Federelemente
- 321: Halteeinrichtung
- 322: Glasspritzenbedieneinrichtungskontaktbereich

- 401: nächstes Ausführungsbeispiel
- 403: Glasspritze
- 404: Montagehilfselement
- 405: Schutzkappenteil
- 408: Spritzenkonusteil
- 409: Spritzennadel
- 414: Außenkontur
- 415: Befestigungseinrichtung
- 416: Innenkontaktbereich
- 418: Abstand
- 419: Nadelspitze
- 420: Federelemente
- 421: Halteeinrichtung
- 422: Glasspritzenbedieneinrichtungskontaktbereich
- 428: Umfangserhöhung
- 430: Schnittstellenelement
- 440: Aufsteckeinrichtung
- 441: Wulst
- 503: Glasspritze
- 504: Montagehilfselement
- 505: Schutzkappenteil
- 508: Spritzenkonusteil
- 509: Spritzennadel
- 512: Schulterbereich
- 550: Verbindungssteg
- 551: Sollbruchstelle

## Patentansprüche

1. Anordnung aus einer Glasspritze (303; 403) und aus einem Montagehilfselement (304; 404), zum Bereitstellen eines Schnittstellenelementes (430) zwischen der Glasspritze (303; 403), welche vorderseitig ein Spritzenkonusteil (408) mit einer Spritzennadel (309; 409) und rückseitig ein Flanschteil umfasst, und einer Glasspritzenbedieneinrichtung (6),
wobei das Montagehilfselement (304; 404) bezogen auf die Spritzennadel (309; 409) vor dem Flanschteil an der Glasspritze (303; 403) derart befestigt ist, dass ein Referenzpunkt des Montagehilfselements (304; 404) immer einen definierten Abstand (318; 418) und die gleiche Lage axial wie auch radial zu der Spritzennadelspitze (319; 419) aufweist,
wobei das Montagehilfselement (304; 404) an einer Außenkontur (314; 414) der Glasspritze (303; 403) angeordnet ist,
**dadurch gekennzeichnet, dass**
das Montagehilfselement (304; 404) an der Glasspritze (303; 403) angespritzt, angeklebt oder angeschweißt angeordnet ist,
wobei das Montagehilfselement (304; 404) eine Befestigungseinrichtung (315) aufweist, mittels der das Montagehilfselement (304; 404) bei einer Anordnung an der Außenkontur (314; 414) der Glasspritze (303; 403) bezogen auf die Spritzennadel (309; 409) vor dem Flanschteil an der Glasspritze (303; 403) derart befestigt ist, dass stets ein definierter Abstand (318; 418) zwischen einem Referenzpunkt des Montagehilfeelements (304; 404) und der Nadelspitze (319; 419) der Spritzennadel (309; 409) ausgebildet ist,
wobei die Befestigungseinrichtung einen Aufnahmebereich zur Aufnahme des Spritzenkonusteils (408) aufweist, mittels welcher das Montagehilfselement (404) an dem Spritzenkonusteil (408) und somit an der Glasspritze (403) befestigt ist, oder wobei die Befestigungseinrichtung (315) einen Aufnahmebereich zur Aufnahme eines Schulterbereichs (312) der Glasspritze (303) aufweist, wobei das Montagehilfselement (304) an dem Schulterbereich (312) und somit an der Glasspritze (303) befestigt ist,
wobei das Montagehilfselement (304; 404) eine außen umfangsseitig an dem Montagehilfselement (304; 404) angeordnete Halteeinrichtung (321; 421) aufweist, mittels welcher das Montagehilfselement (304; 404) an der Glasspritzenbedieneinrichtung (6) gehalten ist, wobei die Halteeinrichtung (321; 421) eine spezifische Außenstruktur in Gestalt einer Nut-Feder-Konstruktion aufweist, wobei diesbezügliche Nuten bzw. Federn in Umfangsrichtung und/oder in Längsrichtung des Montagehilfselements (304; 404) verlaufend angeordnet sind und wobei an der Glasspritzenbedieneinrichtung (6) eine mit der Halteeinrichtung (321; 421) wechselwirkende Gegenstruktur ausgestaltet ist.

2. Anordnung nach Anspruch 1,
**dadurch gekennzeichnet, dass**
die Befestigungseinrichtung eine umlaufende Nut zum Anordnen einer Umfangserhöhung (428) des Spritzenkonusteils (408) aufweist.

3. Anordnung nach einem der Ansprüche 1 oder 2,
**dadurch gekennzeichnet, dass**
das Montagehilfselement (304; 404) einen der Glasspritze (303; 403) zugewandten Innenkontaktbereich (416) und einen der Glasspritze (303; 403) abgewandten Glasspritzenbedieneinrichtungskontaktbereich (322; 422) umfasst, wobei der Innenkontaktbereich (416) weicher ausgestaltet ist als der Glasspritzenbedieneinrichtungskontaktbereich (322; 422).

4. Anordnung nach einem der Ansprüche 1 bis 3,
**dadurch gekennzeichnet, dass**
das Montagehilfselement (304; 404) ein Schutzkappenteil (405) umfasst, welches mittels einer Sollbruchstelle (551) an dem Montagehilfselement (304; 404) angeordnet ist und mittels welchem die Spritzennadel (309; 409) abgedeckt ist.

5. Verfahren zum Befestigen des glasspritzenseitigen Montagehilfselements (404) der Anordnung nach einem der Ansprüche 1 bis 4 an einer Glasspritze (403) nach einem der Ansprüche 1 bis 4,
wobei die Glasspritze (403) vorderseitig das Spritzenkonusteil (408) mit einer daran ausgebildeten Umfangserhöhung (428) umfasst, wobei zwischen dem Spritzenkonusteil (408) und dem Flanschteil der Schulterbereich und ein Zylinderbereich angeordnet sind,
wobei die Befestigungseinrichtung des Montagehilfselements (404) außen an der Glasspritze (403) angebracht wird,
**dadurch gekennzeichnet, dass**
die Befestigungseinrichtung an das Spritzenkonusteil (408) und die Umfangserhöhung (428) angeschweißt wird.

6. Verfahren zum Herstellen des glasspritzenseitigen Montagehilfselements (304; 404) der Anordnung nach einem der Ansprüche 1 bis 4 zum Bereitstellen eines Schnittstellenelementes (430) zwischen der Glasspritze (303; 403) der Anordnung nach einem der Ansprüche 1 bis 4 und der Glasspritzenbedieneinrichtung (6) der Anordnung nach einem der Ansprüche 1 bis 4,
**dadurch gekennzeichnet, dass**
das Montagehilfselement (304; 404) mindestens zweilagig hergestellt wird, wobei es an einer der Glasspritze (303; 403) zugewandten Seite weicher ausgestaltet wird als an einer der Glasspritze (303; 403) abgewandten Seite, an welcher die Glasspritzenbedieneinrichtung (6) angeordnet wird.

## Claims

1. Arrangement consisting of a glass syringe (303; 403) and an assembly auxiliary element (304; 404) for providing an interface element (430) between the glass syringe (303; 403), which comprises, on the front end, a syringe cone part (408) having a syringe needle (309; 409) and, on the rear end, a flange part, and a glass syringe operating means (6), the assembly auxiliary element (304; 404) being secured to the glass syringe (303; 403) in front of the flange part relative to the syringe needle (309; 409) such that a reference point of the assembly auxiliary element (304; 404) is always at a defined distance (318; 418) from the syringe needle tip (319; 419) and in the same axial and radial position relative to said syringe needle tip, the assembly auxiliary element (304; 404) being arranged on an outer contour (314; 414) of the glass syringe (303; 403), **characterised in that** the assembly auxiliary element (304; 404) is injection-moulded, adhered or welded onto the glass syringe (303; 403), the assembly element (304; 404) comprising a securing means (315) by means of which the assembly auxiliary element (304; 404), arranged on the outer contour (314; 414) of the glass syringe (303; 403), is secured to the glass syringe (303; 403) in front of the flange part relative to the syringe needle (309; 409) such that a defined distance (318; 418) is always formed between a reference point of the assembly auxiliary element (304; 404) and the needle tip (319; 419) of the syringe needle (309; 409), the securing means having a receiving area for receiving the syringe cone part (408), by means of which the assembly auxiliary element (404) is secured to the syringe cone part (408) and thus to the glass syringe (403), or the securing means (315) having a receiving area for receiving a shoulder region (312) of the glass syringe (303), the assembly auxiliary element (304) being secured to the shoulder region (312) and thus to the glass syringe (303), the assembly auxiliary element (304; 404) comprising a holding device (321; 421) arranged on the outer peripheral side of the assembly auxiliary element (304; 404), by means of which the assembly auxiliary element (304; 404) is held on the glass syringe operating means (6), the holding device (321; 421) having a specific outer structure in the form of a tongue- and-groove configuration, respective grooves and tongues being arranged such that they extend in the peripheral direction and/or in the longitudinal direction of the assembly auxiliary element (304; 404), and a counter structure which interacts with the holding device (321; 421) being formed on the glass syringe operating means (6).

2. Arrangement according to claim 1, **characterised in that** the securing means comprises a peripheral groove for the arrangement of a peripheral elevation (428) of the syringe cone part (408).

3. Arrangement according to either claim 1 or claim 2, **characterised in that** the assembly auxiliary element (304; 404) comprises an inner contact area (416) which faces the glass syringe (303; 403) and a glass syringe operating means contact area (322; 422) which faces away from the glass syringe (303; 403), the inner contact area (416) being softer than the glass syringe operating means contact area (322; 422).

4. Arrangement according to any of claims 1 to 3, **characterised in that** the assembly auxiliary element (304; 404) comprises a protective cap part (405) which is arranged on the assembly auxiliary element (304; 404) by means of a predetermined breaking point (551), and by means of which the syringe needle (309; 409) is covered.

5. Method for securing the glass syringe-side assembly auxiliary element (404) of the arrangement according to any of claims 1 to 4 to a glass syringe (403) according to any of claims 1 to 4, the glass syringe (403) comprising, on the front end, the syringe cone part (408) having a peripheral elevation (428) formed thereon, the shoulder region and a cylinder region being arranged between the syringe cone part (408) and the flange part, the securing means of the assembly auxiliary element (404) being attached to the outside of the glass syringe (403), **characterised in that** the securing means is welded onto the syringe cone part (408) and the peripheral elevation (428).

6. Method for producing the glass syringe-side assembly auxiliary element (304; 404) of the arrangement according to any of claims 1 to 4 for providing an interface element (430) between the glass syringe (303; 403) of the arrangement according to any of claims 1 to 4 and the glass syringe operating means (6) of the arrangement according to any of claims 1 to 4, **characterised in that** the assembly auxiliary element (304; 404) is produced having at least two layers, said element being softer on a side which faces the glass syringe (303; 403) than on a side which faces away from the glass syringe (303; 403) and on which the glass syringe operating means (6) is arranged.

## Revendications

1. Ensemble formé par une seringue en verre (303 ; 403) et un auxiliaire de montage (304 ; 404), pour fournir un élément d'interface (430) entre la seringue en verre (303 ; 403), laquelle comporte côté avant une partie cône de seringue (408) pourvue d'une aiguille de seringue (309 ; 409) et côté arrière une partie bride, et un dispositif (6) d'actionnement de la seringue en verre,
dans lequel l'auxiliaire de montage (304 ; 404) est fixé par rapport à l'aiguille de seringue (309 ; 409) devant la partie bride sur la seringue en verre (303 ; 403) de telle sorte qu'un point de référence de l'auxiliaire de montage (304 ; 404) est toujours à une distance définie (318 ; 418) et à la même position axialement ainsi que radialement par rapport à la pointe d'aiguille de seringue (319 ; 419),
dans lequel l'auxiliaire de montage (304 ; 404) est disposé sur un contour externe (314 ; 414) de la seringue en verre (303 ; 403),
**caractérisé par le fait que**
l'auxiliaire de montage (304 ; 404) est disposé moulé par injection, collé ou soudé sur la seringue en verre (303 ; 403),
dans lequel l'auxiliaire de montage (304 ; 404) présente un dispositif de fixation (315) au moyen duquel l'auxiliaire de montage (304 ; 404) lors d'un assemblage sur le contour externe (314 ; 414) de la seringue en verre (303 ; 403) est fixé par rapport à l'aiguille de seringue (309 ; 409) devant la partie bride sur la seringue en verre (303 ; 403) de telle sorte qu'une distance définie (318 ; 418) est toujours formée entre un point de référence de l'auxiliaire de montage (304 ; 404) et la pointe d'aiguille (319 ; 419) de l'aiguille de seringue (309 ; 409) ;
dans lequel le dispositif de fixation présente une région de réception pour la réception de la partie cône de seringue (408), au moyen duquel l'auxiliaire de montage (404) est fixé à la partie cône de seringue (408) et par conséquent à la seringue en verre (403), ou dans lequel le dispositif de fixation (315) présente une région de réception pour la réception d'une région d'épaulement (312) de la seringue en verre (303), l'auxiliaire de montage (304) étant fixé sur la région d'épaulement (312) et par conséquent sur la seringue en verre (303) ;
dans lequel l'auxiliaire de montage (304, 404) présente un dispositif de maintien (321 ; 421) disposé sur l'auxiliaire de montage (304 ; 404), côté périphérie externe, au moyen duquel l'auxiliaire de montage (304 ; 404) est maintenu sur le dispositif (6) d'actionnement de la seringue en verre, le dispositif de maintien (321 ; 421) présentant une structure externe spécifique sous la forme d'une structure rainure-languette, à ce sujet des rainures ou des languettes étant disposées s'étendant dans la direction périphérique et/ou dans la direction longitudinale de l'auxiliaire de montage (304 ; 404), et une contre-structure interagissant avec le dispositif de maintien (321 ; 421) étant agencée sur le dispositif (6) d'actionnement de la seringue en verre.

2. Ensemble selon la revendication 1,
**caractérisé par le fait que**
le dispositif de fixation présente une rainure périphérique pour disposer une élévation périphérique (428) de la partie cône de seringue (408).

3. Ensemble selon l'une des revendications 1 ou 2,
**caractérisé par le fait que**
l'auxiliaire de montage (304 ; 404) comporte une région de contact interne (416) tournée vers la seringue en verre (303 ; 403) et une région (322 ; 422) de contact de dispositif d'actionnement de seringue en verre tourné à l'opposé de la seringue en verre (303 ; 403), la région de contact interne (416) étant conçue pour être plus souple que la région (322 ; 422) de contact de dispositif d'actionnement de seringue en verre.

4. Ensemble selon l'une des revendications 1 à 3,
**caractérisé par le fait que**
l'auxiliaire de montage (304 ; 404) comporte une partie capuchon protecteur (405), laquelle est disposée sur l'auxiliaire de montage (304 ; 404) au moyen d'une zone de rupture (551) et au moyen de laquelle l'aiguille de seringue (309 ; 409) est recouverte.

5. Procédé pour la fixation de l'auxiliaire de montage (404) côté seringue en verre de l'ensemble selon l'une des revendications 1 à 4 sur une seringue en verre (403) selon l'une des revendications 1 à 4,
dans lequel la seringue en verre (403) comporte côté avant la partie cône de seringue (408) avec une élévation périphérique (428) formée sur celle-ci, la région d'épaulement et une région cylindrique étant disposées entre la partie cône de seringue (408) et la partie bride,
dans lequel le dispositif de fixation de l'auxiliaire de montage (404) est fixé extérieurement sur la seringue en verre (403),
**caractérisé par le fait que**
le dispositif de fixation est soudé sur la partie cône de seringue (408) et l'élévation périphérique (428).

6. Procédé de fabrication de l'auxiliaire de montage (304 ; 404) côté seringue en verre de l'ensemble selon l'une des revendications 1 à 4 pour fournir un élément d'interface (430) entre la seringue en verre (303 ; 403) de l'ensemble selon l'une des revendications 1 à 4 et le dispositif (6) d'actionnement de la seringue en verre de l'ensemble selon l'une des revendications 1 à 4,
**caractérisé par le fait que**
l'auxiliaire de montage (304 ; 404) est fabriqué au moins en deux couches, à l'occasion de quoi il est conçu sur un côté tourné vers la seringue en verre (303 ; 403) plus souple que sur un côté tourné à l'opposé à la seringue en verre (303 ; 403) sur lequel le dispositif (6) d'actionnement de la seringue en verre est disposé.
